(19) Europäisches Patentamt
European Patent Office
Office européen des brevets

(11) Numéro de publication : **0 028 179**
**B1**

(12) **FASCICULE DE BREVET EUROPÉEN**

(45) Date de publication du fascicule du brevet : **03.11.82**

(51) Int. Cl.³ : **C 07 C120/00**, C 07 C121/46

(21) Numéro de dépôt : **80401455.3**

(22) Date de dépôt : **10.10.80**

(54) **Procédé de préparation de cyano-3-triméthyl-3,5,5-cyclohexanone.**

(30) Priorité : **26.10.79 FR 7926596**

(43) Date de publication de la demande : **06.05.81 (Bulletin 81/18)**

(45) Mention de la délivrance du brevet : **03.11.82 Bulletin 82/44**

(84) Etats contractants désignés : **AT BE CH DE FR GB IT LI LU NL SE**

(56) Documents cités :
**FR A 1 452 374**
**US A 3 974 199**

**CHEMICAL ABSTRACTS, vol. 84, no. 17, 26 avril 1976, résumé 120128h, page 477, COLUMBUS, OHIO (US)**

(73) Titulaire : **P C U K PRODUITS CHIMIQUES UGINE KUHLMANN**
**Service Propriété Industrielle Tour Manhattan**
**F-92087 Paris La Défense 2 Cedex 21 (FR)**

(72) Inventeur : **Dubreux, Bernard**
**29 Avenue du Chater Bâtiment A 3**
**F-69340 Francheville Le Bas (FR)**

**EP 0 028 179 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation de cyano-3-triméthyl-3,5,5-cyclohexanone

Les β-cyanocétones sont des intermédiaires importants dans la préparation des δ-diamines primaires, elles-mêmes utilisées notamment dans la synthèse de polyamides et comme catalyseurs de durcissement des résines époxy. La présente invention, qui concerne un procédé de préparation d'une β-cyanocétone particulière, la cyano-3-triméthyl-3,5,5-cyclohexanone, à partir d'isophorone et de cyanures, se caractérise par la mise en œuvre dans la synthèse d'un système à deux phases et d'un agent de transfert de phase utilisé en quantité catalytique.

L'action de l'acide cyanhydrique et des cyanures sur les cétones α,β-insaturées est décrite. Toutefois, si l'on utilise les cyanures la réaction est lente, les temps de contacts sont élevés et on obtient des rendements faibles. Si l'on utilise l'acide cyanhydrique les rendements sont bons mais la mise en œuvre du procédé est délicate. En effet, les températures de réaction sont élevées ce qui contraint le plus souvent à opérer sous pression. De plus, la réaction nécessitant une catalyse basique, de grandes précautions doivent être prises pour éviter la polymérisation très exothermique de l'acide cyanhydrique.

Le brevet français N° 1 452 374 décrit un procédé de fabrication de la cyano-3-triméthyl-3,5,5-cyclohexanone par réaction en phase homogène de l'isophorone avec l'acide cyanhydrique en présence de très faibles quantités d'un catalyseur alcalin. Malgré l'emploi de quantités réduites de ce catalyseur alcalin il subsiste un risque de polymérisation violente de l'acide cyanhydrique.

Or la demanderesse vient de découvrir que l'on pouvait synthétiser la cyano-3-triméthyl-3,5,5-cyclohexanone avec de bonnes sélectivités et dans des conditions douces à partir de l'isophorone et de cyanures. Pour la mise en œuvre du procédé selon l'invention on oppose une phase aqueuse contenant le cyanure à une phase organique contenant l'isophorone en présence d'une quantité catalytique d'un agent de transfert de phase. On réalise alors formellement la réaction suivante :

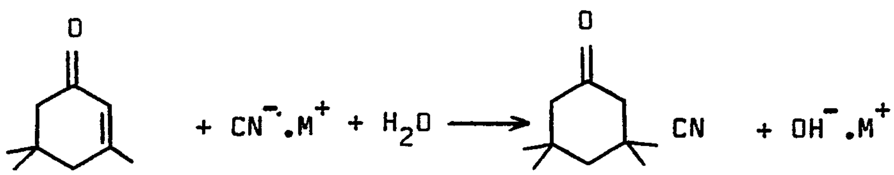


La catalyse par un agent de transfert de phase de réactions entre un réactif dissous dans une phase aqueuse et un réactif dissous dans une phase organique a déjà été appliquée à des réactions d'élimination intramoléculaire. C'est ainsi que le brevet des Etats-Unis N° 3 974 199 décrit un procédé de préparation du cyanure de cyclopropyle par réaction d'un γ-halobutyronitrile avec un hydroxyde de métal alcalin en présence d'au moins une trace d'eau et d'une quantité catalytique d'un sel onium. Mais cette catalyse n'a pas été appliquée jusqu'ici à la réaction d'addition d'ions cyanure sur la double liaison conjuguée d'une cétone éthylénique.

La phase organique utilisable selon l'invention peut être soit une solution d'isophorone dans un solvant, soit l'isophorone elle-même. La phase aqueuse est obtenue par exemple par dissolution d'un cyanure dans l'eau ou par neutralisation d'une base par l'acide cyanhydrique. Après avoir ajouté un agent de transfert en quantité catalytique on met les deux phases en présence dans un réacteur muni d'une bonne agitation et on maintient l'ensemble à la température désirée.

Les solvants utilisables selon l'invention sont choisis uniquement en fonction de la température de réaction et des caractéristiques physiques de l'isophorone et de la cyano-3-triméthyl-3,5,5-cyclohexanone — points de fusion, solubilité, points d'ébullition — à l'exclusion de tous les solvants miscibles à l'eau dans les conditions de la réaction ou susceptibles de réagir avec une solution aqueuse alcaline de cyanures. Parmi les solvants susceptibles d'être utilisés on retiendra les hydrocarbures, les éthers, les esters, les alcools lourds, etc. La concentration de l'isophorone dans la phase organique peut être comprise entre 0,1 % en poids et la saturation à la température de la réaction. On peut utiliser un mélange de plusieurs solvants.

Les cyanures utilisables dans la phase aqueuse sont tous les cyanures solubles dans l'eau à raison de plus de 0,1 % en poids. On utilisera par exemple les cyanures de potassium ou de sodium. La concentration en cyanures dans la phase aqueuse peut être comprise entre 0,1 % en poids et la saturation à la température de la réaction.

Le rapport molaire isophorone/cyanure peut varier dans de grandes proportions. Il dépendra essentiellement du rapport des volumes de la phase aqueuse et de la phase organique engagées et des solubilités respectives des cyanures et de l'isophorone. Le plus souvent ce rapport est compris entre 0,01 et 10 et préférentiellement entre 0,1 et 1.

Les agents de transfert de phase utilisables selon la présente invention sont préférablement des sels d'ammonium ou de phosphonium quaternaires solubles dans la phase organique à raison de plus de 0,01 % en poids et qui sont par conséquent choisis en fonction de la nature de la phase organique mise en œuvre. Le rapport molaire catalyseur/isophorone pourra être compris entre 0,000 01 et 0,05 et préférentiellement entre 0,000 5 et 0,02. Parmi les agents de transfert utilisables selon l'invention on peut mentionner les sels de benzyltriméthylammonium, benzyltriéthylammonium, tétraméthylammonium,

tétraéthylammonium, tétrabutylammonium, dodécényltriéthylammonium, lauryldiméthylbenzylammonium, etc. et les sels de tétraéthylphosphonium, tétrabutylphosphonium, lauryltriéthylphosphonium, lauryltributylphosphonium, tétraphénylphosphonium, etc.

La température de la réaction est le plus souvent comprise entre 0 °C et 100 °C et préférentiellement entre 60 °C et 90 °C.

On peut réaliser le procédé de façon continue ou discontinue.

Au fur et à mesure de l'avancement de la réaction l'alcalinité de la phase aqueuse augmente, comme l'indique l'équation chimique. On peut, si on le désire, limiter cette augmentation d'alcalinité par adjonction de réactifs tampons ou bien en additionnant au fur et à mesure au mélange un acide. On utilisera par exemple l'acide chlorhydrique, l'acide sulfurique ou l'acide phosphorique. On peut également utiliser avantageusement l'acide cyanhydrique qui présente l'avantage de régénérer le cyanure consommé par la réaction.

Les exemples suivants illustrent de façon non limitative la présente invention.

Exemple 1

On met en contact une phase organique constituée par 0,360 mole d'isophorone et une phase aqueuse constituée par une solution de 0,5 mole de cyanure de sodium dans 35 g d'eau. On chauffe à 80 °C et introduit 1 g de bromure de lauryldiméthylbenzylammonium (en abrégé : BLDMBA). Après un temps de réaction de 4 heures à 80 °C, l'analyse chromatographique en phase vapeur montre qu'il s'est formé 0,087 mole de cyano-3-triméthyl-3,5,5-cyclohexanone. La sélectivité, exprimée par le rapport molaire de la cyano-cétone formée à l'isophorone transformée, atteint 90 %. La cyano-3-triméthyl-3,5,5-cyclohexanone est isolée du milieu réactionnel, soit par distillation à 146-148 °C sous 16 mm de mercure, soit par cristallisation.

Exemple 2

Dans cet exemple, la phase organique est constituée par une solution de 0,360 mole d'isophorone dans 80 g d'alcool n-amylique, et la phase aqueuse est constituée par une solution de 0,25 mole de cyanure de sodium dans 50 g d'eau. Après chauffage à 70 °C, on introduit 1 g de bromure de tétraéthylammonium et laisse réagir pendant 5 heures à 70 °C. On obtient 0,057 mole de cyano-cétone avec une sélectivité de 93 %.

Exemple 3

On opère comme à l'exemple 2, mais en utilisant une solution de 0,1 mole d'isophorone dans 22 g d'alcool n-amylique et une solution de 0,7 mole de cyanure de sodium dans 70 g d'eau. Il se forme 0,038 mole de cyano-cétone, avec une sélectivité de 80 %.

Exemple 4

On met en contact une solution de 0,05 mole d'isophorone dans 24 g de ligroïne et une solution de 1 mole de cyanure de sodium et 0,05 mole de phosphate disodique dans 100 g d'eau. On chauffe à 80 °C et introduit 0,5 g de BLDMBA. L'agent tampon maintient le pH de la solution aqueuse aux environs de 12,5. Après 2 heures de réaction à 80 °C, il s'est formé 0,022 mole de cyano-cétone, avec une sélectivité de 81 %.

Exemple 5

On opère comme à l'exemple 4, mais en utilisant 0,1 mole d'isophorone en solution dans 24 g de ligroïne et 1 mole de cyanure de sodium en solution dans 150 g d'eau. Le tampon, constitué par 0,15 mole de phosphate disodique et 0,03 mole de soude, maintient le pH aux environs de 12,5. Après 4 heures de réaction, il s'est formé 0,048 mole de cyano-cétone, avec une sélectivité de 78 %.

Exemple 6

On met en contact une solution de 0,2 mole d'isophorone dans 7 g d'alcool n-amylique avec une solution de 1 mole de cyanure de sodium dans 100 g d'eau. Après chauffage à 80 °C, on introduit 1 g de BLDMBA, puis on coule une solution aqueuse à 10 % d'acide cyanhydrique, de façon à maintenir le pH aux environs de 11,5. Après 3 heures et demie de réaction à 80 °C, il s'est formé 0,141 mole de cyano-cétone, avec une sélectivité de 92 %.

Exemple 7

On opère comme à l'exemple 6, mais en utilisant une solution de 0,5 mole d'isophorone dans un

mélange de 100 g de ligroïne et 14 g d'alcool n-amylique, et une solution de 2 moles de cyanure de sodium dans 200 g d'eau. Le BLDMBA est utilisé à la dose de 2 g et le pH est réglé aux environs de 11,5 par coulée d'une solution aqueuse à 30 % d'acide cyanhydrique. Après 5 heures de réaction à 80 °C, il s'est formé 0,320 mole de cyano-cétone, avec une sélectivité de 98 %.

## Revendications

1. Procédé de préparation de la cyano-3-triméthyl-3,5,5-cyclohexanone à partir d'isophorone et de cyanures caractérisé par le fait que l'on met en contact une phase organique contenant l'isophorone et une phase aqueuse contenant le cyanure en présence d'une quantité catalytique d'un agent de transfert de phase.

2. Procédé selon la revendication 1 dans lequel l'isophorone mise en œuvre constitue la phase organique.

3. Procédé selon la revendication 1 dans lequel l'isophorone mise en œuvre est dissoute dans un solvant ou un mélange de solvants à une concentration comprise entre 0,1 % en poids et la saturation à la température de la réaction.

4. Procédé selon la revendication 1 dans lequel les ions cyanures sont en phase aqueuse à une concentration comprise entre 0,1 % en poids et la saturation à la température de la réaction.

5. Procédé selon la revendication 1 dans lequel le cyanure mis en œuvre est du cyanure de potassium.

6. Procédé selon la revendication 1 dans lequel le cyanure mis en œuvre est du cyanure de sodium.

7. Procédé selon les revendications 1 à 6 dans lequel le rapport molaire isophorone/cyanure est compris entre 0,01 et 10 et préférentiellement entre 0,1 et 1.

8. Procédé selon les revendications 1 à 7 dans lequel l'agent de transfert de phase est un sel d'ammonium ou de phosphonium quaternaire soluble dans la phase organique à raison de plus de 0,01 % en poids.

9. Procédé selon la revendication 8 dans lequel l'agent de transfert de phase est le bromure de lauryldiméthylbenzylammonium.

10. Procédé selon la revendication 8 dans lequel l'agent de transfert est le bromure de tétraéthylammonium.

11. Procédé selon les revendications 1 à 10 dans lequel le rapport molaire catalyseur/isophorone est compris entre 0,000 01 et 0,05 et préférentiellement entre 0,000 5 et 0,02.

12. Procédé selon les revendications 1 à 11 dans lequel la température de réaction est comprise entre 0 °C et 100 °C et de préférence entre 60 °C et 90 °C.

13. Procédé selon l'une des revendications 1 à 12 dans lequel on limite l'augmentation d'alcalinité par adjonction d'un réactif tampon ou d'un acide.

14. Procédé selon la revendication 13 dans lequel on ajoute du phosphate disodique.

15. Procédé selon la revendication 13 dans lequel on ajoute de l'acide chlorhydrique.

16. Procédé selon la revendication 13 dans lequel on ajoute de l'acide sulfurique.

17. Procédé selon la revendication 13 dans lequel on ajoute de l'acide cyanhydrique.

## Claims

1. Process for the preparation of 3-cyano-3,5,5-trimethylcyclohexanone from isophorone and cyanides, characterised in that an organic phase containing the isophorone and an aqueous phase containing a cyanide are brought into contact in the presence of a catalytic amount of a phase transfer agent.

2. Process according to Claim 1, in which the isophorone employed constitutes the organic phase.

3. Process according to Claim 1, in which the isophorone employed is dissolved in a solvent or a mixture of solvents at a concentration of between 0.1 % by weight and the saturation concentration at the reaction temperature.

4. Process according to Claim 1, in which the cyanide ions are present in the aqueous phase at a concentration of between 0.1 % by weight and the saturation concentration at the reaction temperature.

5. Process according to Claim 1, in which the cyanide employed is potassium cyanide.

6. Process according to Claim 1, in which the cyanide employed is sodium cyanide.

7. Process according to Claims 1 to 6, in which the molar ratio of isophorone to cyanide is between 0.01 and 10 and preferably between 0.1 and 1.

8. Process according to Claims 1 to 7, in which the phase transfer agent is a quaternary ammonium or quaternary phosphonium salt which is soluble in the organic phase to the extent of more than 0.01 % by weight.

9. Process according to Claim 8, in which the phase transfer agent is lauryldimethylbenzylammonium bromide.

10. Process according to Claim 8, in which the transfer agent is tetraethylammonium bromide.

11. Process according to Claims 1 to 10, in which the molar ratio of catalyst to isophorone is between 0.000 01 and 0.05 and preferably between 0.000 5 and 0.02.

12. Process according to Claims 1 to 11, in which the reaction temperature is between 0 °C and 100 °C and preferably between 60 °C and 90 °C.

13. Process according to one of Claims 1 to 12, in which the increase in alkalinity is restricted by addition of a buffer reagent or of an acid.

14. Process according to Claim 13, in which disodium phosphate is added.

15. Process according to Claim 13, in which hydrochloric acid is added.

16. Process according to Claim 13, in which sulphuric acid is added.

17. Process according to Claim 13, in which hydrocyanic acid is added.

**Ansprüche**

1. Verfahren zur Herstellung von 3-Cyan-3,5,5-trimethylcyclohexanon aus Isophoron und Cyaniden, dadurch gekennzeichnet, daß man eine organische Phase, die Isophoron enthält, und eine wässrige Phase, die das Cyanid enthält, in Gegenwart einer katalytischen Menge eines Phasenübertragungsmittels in Berührung bringt.

2. Verfahren nach Anspruch 1, bei dem das eingesetzte Isophoron die organische Phase bildet.

3. Verfahren nach Anspruch 1, bei dem das eingesetzte Isophoron in einem Lösungsmittel oder einem Lösungsmittelgemisch in einer Konzentration zwischen 0,1 Gew.% und der Sättigung bei der Reaktionstemperatur gelöst wird.

4. Verfahren nach Anspruch 1, bei dem die Cyanide in der wässrigen Phase in einer Konzentration zwischen 0,1 Gew.% und der Sättigung bei der Reaktionstemperatur vorliegen.

5. Verfahren nach Anspruch 1, bei dem das eingesetzte Cyanid Kaliumcyanid ist.

6. Verfahren nach Anspruch 1, bei dem das eingesetzte Cyanid Natriumcyanid ist.

7. Verfahren nach den Ansprüchen 1 bis 6, bei dem das Molverhältnis Isophoron/Cyanid zwischen 0,01 und 10, vorzugsweise zwischen 0,1 und 1 liegt.

8. Verfahren nach den Patentansprüchen 1 bis 7, bei dem das Phasenübertragungsmittel ein in der organischen Phase zu mehr als 0,01 Gew.% lösliches quaternäres Ammonium- oder Phosphoniumsalz ist.

9. Verfahren nach Anspruch 8, bei dem das Phasenübertragungsmittel Lauryldimethylbenzylammoniumbromid ist.

10. Verfahren nach Anspruch 8, bei dem das Phasenübertragungsmittel Tetraäthylammoniumbromid ist.

11. Verfahren nach den Ansprüchen 1 bis 10, bei dem das Molverhältnis Katalysator/Isophoron zwischen 0,000 01 und 0,05, vorzugsweise zwischen 0,000 5 und 0,02 liegt.

12. Verfahren nach den Ansprüchen 1 bis 11, bei dem die Reaktionstemperatur zwischen 0 und 100, vorzugsweise zwischen 60 und 90 °C liegt.

13. Verfahren nach einem der Ansprüche 1 bis 12, bei dem man die Erhöhung der Alkalinität durch Zugabe eines Puffers oder einer Säure begrenzt.

14. Verfahren nach Anspruch 13, bei dem man Dinatriumphosphat zugibt.

15. Verfahren nach Anspruch 13, bei dem man Salzsäure zugibt.

16. Verfahren nach Anspruch 13, bei dem man Schwefelsäure zugibt.

17. Verfahren nach Anspruch 13, bei dem man Cyanwasserstoffsäure zugibt.